# EUROPÄISCHE PATENTANMELDUNG

(11) **EP 3 650 116 A1**
(43) Veröffentlichungstag der Anmeldung: **13.05.2020**
(21) Anmeldenummer: 19207756.8
(22) Anmeldetag: 07.11.2019
(51) Int. Cl.: B01J 12/00, C07C 29/152, C07C 1/12, B01J 8/02, B01J 8/00, B01J 19/24

(54) **KATALYTISCHER DURCHGANGSMEMBRANREAKTOR ZUR DURCHFÜHRUNG CHEMISCHER GLEICHGEWICHTSREAKTIONEN**

(30) Priorität: 09.11.2018 DE 202018106378 U
(71) Anmelder: MUW SCREENTEC Filter- und Präzisionstechnik aus Metall GmbH, 29378 Wittingen (DE)
(72) Erfinder: GRÜTZNER, Jörg, 16552 Schildow (DE); MARTIN, Dirk, 99085 Erfurt (DE)
(74) Vertreter: Oehmke, Volker

(57) **Zusammenfassung**

Die Erfindung betrifft einen katalytischen Durchgangsmembranreaktor zur Durchführung chemischer Gleichgewichtsreaktionen, insbesondere der Methan- und Methanolsynthese aus Wasserstoff und Kohlendioxid.

Der Erfindung liegt die Aufgabe zugrunde, eine Vorrichtung zur Verfahrensführung von Gleichgewichtsreaktionen vorzuschlagen, bei denen auf der Seite der Reaktionsprodukte Wasser abgetrennt wird und eine verbesserte Ausbeute an Reaktionsprodukten erzielt wird.

Die Aufgabe wird dadurch gelöst, dass ein katalytischen Durchgangsmembranreaktor zur Durchführung chemischer Gleichgewichtsreaktionen aus einem Reaktorblock (1) mit einem Reaktionsraum (2) besteht, wobei dem Reaktorblock (1) ein lösbarer Eingangsblock (3) mit einem Zugang (3.1) vorgeordnet ist und ein gegenüberliegender Ausgangsblock (4) mit einem Abgang (4.1) nachgeordnet ist. Der Reaktorblock (1) weist dabei in seinem Inneren ein Membranrohr (5) mit innenliegender Katalysatorschüttung (6) auf, wobei das Membranrohr (5) durch den Reaktionsraum (2) geführt ist und den Zugang (3.1) mit dem Abgang (4.1) über je eine Eindichtung (7,8) verbindet. Zur Prozessdurchführung weist der Reaktionsraum (2) zur Aufnahme von Stickstoff einen Gaseinlass (12) und zur Abführung von Wasser und Stickstoff ein Auslassrohr (13) auf. Ferner ist im Abgang (4.1) ein Vordruckregler angeordnet.

## Beschreibung

Die Erfindung betrifft einen katalytischen Durchgangsmembranreaktor zur Durchführung chemischer Gleichgewichtsreaktionen, insbesondere der Methan- und Methanolsynthese aus Wasserstoff und Kohlendioxid.

Die Umsetzung von Wasserstoff mit Kohlendioxid wird im Wesentlichen aus zwei Gründen verfolgt. Einerseits wird zunehmend Wasserstoff durch Elektrolyse hergestellt, um Netzschwankungen aus dem zunehmenden Anteil fluktuierenden Stroms aus regenerativen Quellen zu puffern ("power to gas"). Die Speicherung und die Verteilung von Wasserstoff können zu geringen Anteilen direkt im Erdgasnetz erfolgen. Durch die Umsetzung mit Kohlendioxid zu Methan wären die Speicherung und Verteilung in viel größerem Maßstab möglich. Der zweite Grund betrifft die chemische Bindung und Nutzung von Kohlendioxid und hat damit erhebliche umwelt- und klimatechnische Vorteile.

In der dem Fachmann bekannten Sabatier-Reaktion, die eine chemische Gleichgewichtsreaktion ist, werden Kohlendioxid (CO₂) und Wasserstoff (H₂) (Edukte) zu Methan (CH₄) und Wasser (H₂O) (Produkte) umgesetzt. Die Sabatier-Reaktion wird meist bei Drücken von ∼1...3 bara (absoluter Druck; bar absolut), vereinzelt auch bei höheren Drücken von bis zu 15 bara, und in einem Temperaturbereich von rund 200 bis 450 °C durchgeführt. Als geeignete Katalysatoren für diese Reaktion sind beispielsweise Nickel, Ruthenium, Rhodium und Cobalt bekannt, die meist auf einem oxidischen Träger wie SiO₂, TiO₂, MgO, Al₂O₃, La₂O₃ aufgebracht sind, wie dies beispielsweise in Ohya et al. 1997: Methanation of carbon dioxide by using membrane reactor integrated with water vapor permselective membrane and its analysis, Journal of Membrane Science 131 (1-2), beschrieben ist. Am erfolgreichsten stellt sich wohl momentan die Kombination 0,5 % Ru auf TiO₂ dar. Abhängig vom Katalysator können hohe CO₂-Umsätze und CH₄-Ausbeuten bei Temperaturen von ∼300...350°C erreicht werden (John Ralston (http://www.pennenergy.com/index/power /display/0723256773/articles/pennenergy/ugc/renewable/the-sabatier-reaction.html).

Die Sabatier-Reaktion ist eine sehr anspruchsvolle Reaktion, da sie stark exotherm ist und nach Zugabe der Edukte, je nach Verdünnungsgrad mit inerten Gasen, zu Temperaturen über 600 °C führt. Dieses Temperaturniveau zerstört nicht nur manche Katalysatoren, beispielsweise gehen aktive Zentren verloren, indem z. B. Ni-Partikel im nm-Bereich zusammensintern, sondern verschiebt auch das Reaktionsgleichgewicht in Richtung der Edukte. Zu niedrige Temperaturen haben dagegen kinetische Limitierungen der Reaktion und der Menge der gewünschten Produkte zur Folge.

Da es sich bei der Sabatier-Reaktion um eine exotherme Gleichgewichtsreaktion handelt, ist sie mit zunehmender Reaktions- bzw. Prozesstemperatur ausbeutelimitiert. Der Entzug eines Reaktionsproduktes direkt aus der Reaktion im Prozess bewirkt eine Erhöhung der Triebkraft zur Produktbildung. Eine geeignete wasserabtrennende Membran bewirkt genau dies, indem das Reaktionsprodukt Wasser direkt im Prozess abgetrennt wird. So wird eine Ausbeutesteigerung verglichen zum Prozess ohne Membran erreicht. Weiterhin wird durch die Abtrennung des Reaktionswassers eine nachgeordnete Trocknung des Methans zur Erreichung der Einspeisequalität überflüssig und dieser Prozessschritt kann eingespart werden.

Der Erfindung liegt die Aufgabe zugrunde, eine Vorrichtung zur Verfahrensführung von Gleichgewichtsreaktionen vorzuschlagen, bei denen auf der Seite der Reaktionsprodukte Wasser abgetrennt wird und eine verbesserte Ausbeute an Reaktionsprodukten erzielt wird.

Die Durchführung gekoppelter chemischer Reaktion unter selektiver Abtrennung eines Reaktionsproduktes unter drastischen Reaktionsbedingungen (> 200 °C, > 10 bar) bedingt den Einsatz spezieller Lösungen bzgl. der eingesetzten Membranen, deren Eindichtung zur Trennung der Gasräume sowie der Gas- und Temperaturführung.

Die Aufgabe wird dadurch gelöst, dass ein katalytischen Durchgangsmembranreaktor zur Durchführung chemischer Gleichgewichtsreaktionen, insbesondere der Methan- oder Methanolsynthese, aus einem Reaktorblock mit einem Reaktionsraum besteht, wobei dem Reaktorblock ein lösbarer Eingangsblock mit einem Zugang vorgeordnet ist und ein gegenüberliegender Ausgangsblock mit einem Abgang nachgeordnet ist. Der Reaktorblock weist dabei in seinem Inneren ein Membranrohr mit innenliegender Katalysatorschüttung auf, wobei das Membranrohr durch den Reaktionsraum geführt ist und den Zugang mit dem Abgang über je eine Eindichtung verbindet. Zur Prozessdurchführung weist der Reaktionsraum zur Aufnahme von Stickstoff einen Gaseinlass und zur Abführung von Wasser und Stickstoff ein Auslassrohr auf. Ferner ist im Abgang ein Vordruckregler angeordnet.
Für das notwendige Temperaturmanagement sind drei den Reaktionsraum umschließende Ringkammern mit je einem Eingang und Ausgang zum Heizen oder Kühlen des Reaktionsraumes vorhanden.

Eine weitere zweckmäßige Ausführung ergibt sich, wenn ein Membranrohr zum Einsatz kommt, welches mit einem Katalysator beschichtet ist. Somit würde der erfindungsgemäße Durchgangsmembranreaktor keine Katalysatorschicht mehr bedürfen.

Die Erfindung soll nachfolgend anhand von einem Ausführungsbeispiel erläutert werden. In den dazugehörigen Zeichnungen zeigen:

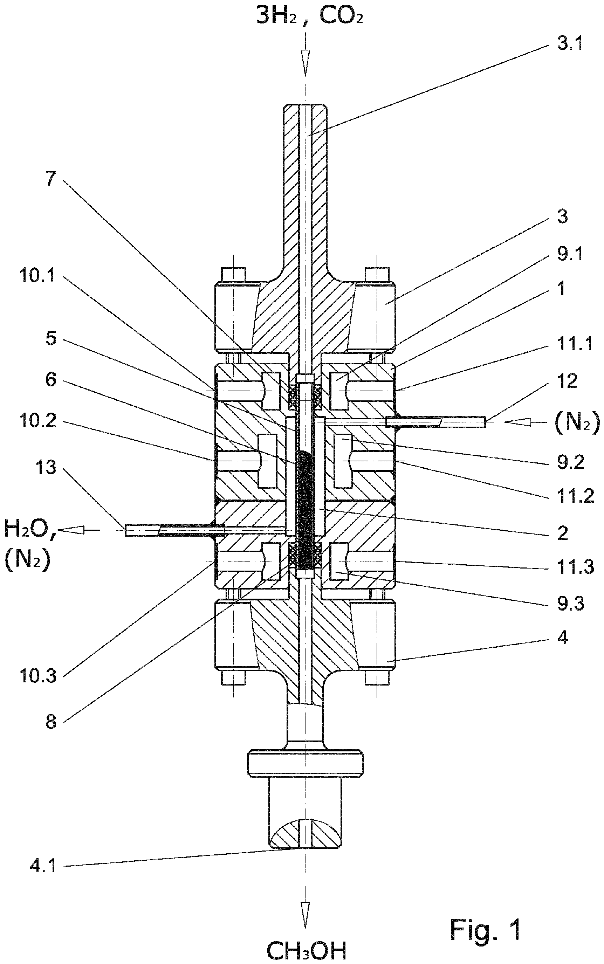

Fig. 1 eine Prinzipdarstellung eines katalytischen Durchgangsmembranreaktors.

Der katalytische Durchgangsmembranreaktor bestehend aus einem Reaktorblock 1, einem Eingangsblock 3 und einem Ausgangsblock 4 ist in Fig. 1 dargestellt. Im Inneren des Reaktorblocks 1 befindet sich der Reaktionsraum 2 durch den das Membranrohr 5 verläuft und den Zugang 3.1 mit dem Abgang 4.1 verbindet. Das Membranrohr 5 ist dabei auf der einen Seite über eine Eindichtung 7 mit dem Eingangsblock 3 und auf der anderen Seite über eine weitere Eindichtung 8 mit dem Ausgangsblock 4 verbunden. Das Membranrohr ist mit einer Katalysatorschüttung 8 befüllt. Der Eingangsblock 3 und der Ausgangsblock 4 sind mit dem Reaktorblock 1 lösbar verbunden, damit das Membranrohr 5 und die Katalysatorschüttung 6 im Reaktionsraum 2 positioniert werden können. Für das Temperaturmanagement sind gemäß Fig.1 drei Ringkammern 9.1, 9.2, 9.3 mit den jeweiligen Eingängen 10.1, 10.2, 10.3 und Ausgängen 11.1, 11.2, 11.3 zu erkennen.

Das im passenden Verhältnis aus Kohlendioxid und Wasserstoff bereitete Eduktgas wird durch den Zugang 3.1 hindurch in das Innere des Membranrohres 5 geleitet, so dass das Eduktgas gleichmäßig verteilt der Katalysatorschüttung 6 zugeführt wird. Sodann erfolgt die Umsetzung des Reaktionsgases zum Produktgas welches den Reaktionsraum 3 durch das Auslassrohr 13 verlässt. Das mittels den Eindichtungen 7,8 implementierte Membranrohr 5 trennt selektiv eine signifikante Menge Wasserdampf als Teil des entstehenden Produktgas ab, welcher durch das Auslassrohr 13 ebenfalls den Reaktionsraum 2 verlässt. Für einen optimalen Prozessablauf wir Stickstoff über den Gaseinlass 12 in den Reaktionsraum geführt, welches danach über das Auslassrohr 13 wieder abgeführt wird. Hierdurch wird das chemische Gleichgewicht der Reaktionen beeinflusst und die Katalysatorschüttung 6 zu höheren Umsätzen des Reaktionsgases bzw. zur Bildung größerer Mengen des Produktgases befähigt. Mittels Fluide, die über die Eingänge 10.1, 10.2, 10.3 der Ringkammern 9.1, 9.2, 9.3 zugeführt werden und über die Ausgänge 11.1, 11.2, 11.3 den Reaktor wieder verlassen kann ein Heizen und Kühlen des Reaktionsraumes 2 ermöglicht werden. Erwähnt sei noch, dass für den Prozessablauf ein Vordruckregler vorhanden sein muss, der zweckmäßiger Weise im Abgang 4.1 angeordnet wird. Ferner ist in einer weiteren Ausführungsform das Membranrohr 5 mit einer Katalysatorschicht versehen. Bei entsprechender Dimensonierung kann dadurch auch ganz auf die Katalysatorschicht verzichtet werden.

### Bezugszeichenliste

- 1: Reaktorblock
- 2: Reaktionsraum
- 3: Eingangsblock
- 3.1: Zugang
- 4: Ausgangsblock
- 4.1: Abgang
- 5: Membranrohr
- 6: Katalysatorschüttung
- 7: Eindichtung
- 8: Eindichtung
- 9.1: Ringkammer
- 9.2: Ringkammer
- 9.3: Ringkammer
- 10.1: Eingang
- 10.2: Eingang
- 10.3: Eingang
- 11.1: Ausgang
- 11.2: Ausgang
- 11.3: Ausgang
- 12: Gaseinlass
- 13: Auslassrohr

## Patentansprüche

1. Katalytischer Durchgangsmembranreaktor zur Durchführung chemischer Gleichgewichtsreaktionen, insbesondere der Methan- oder Methanolsynthese, bestehend aus:
- einem Reaktorblock (1) mit einem Reaktionsraum (2), der durch einen vom Reaktorblock (1) lösbaren Eingangsblock (3) mit einem Zugang (3.1) und einen gegenüberliegenden Ausgangsblock (4) mit einem Abgang (4.1) verschlossen ist,
- einem Membranrohr (5) mit innenliegender Katalysatorschüttung (6), wobei das Membranrohr (5) durch den Reaktionsraum (2) geführt ist und am Zugang (3.1) und am Abgang (4.1) je eine Eindichtung (7, 8) aufweist,
- drei den Reaktionsraum (2) umschließenden Ringkammern (9.1, 9.2, 9.3) mit je einem Eingang (10.1, 10.2, 10.3) und Ausgang (11.1, 11.2, 11.3) zum Heizen oder Kühlen des Reaktionsraumes (2), wobei
- der Reaktionsraum (2) zur Aufnahme von Stickstoff einen Gaseinlass (12) und zur Abführung von Wasser und Stickstoff ein Auslassrohr (13) aufweist und
- im Abgang (4.1) ein Vordruckregler vorhanden ist.

2. Katalytischer Durchgangsmembranreaktor nach Anspruch 1, **dadurch gekennzeichnet, dass** der Reaktorblock (1) aus zwei Teilen zusammen gefügt ist.

3. Katalytischer Durchgangsmembranreaktor nach Anspruch 1 oder 2, **dadurch gekennzeichnet, dass** das Membranrohr (5) mit einer Katalysatorschicht versehen ist.
